# EUROPEAN PATENT APPLICATION

(11) **EP 0 770 616 A1**
(43) Date of publication of application: **02.05.1997**
(21) Application number: 95116932.5
(22) Date of filing: 27.10.1995
(51) Int. Cl.: C07D 493/10, C12P 17/18, A61K 31/34

(54) **Papyracillic acid, method for preparation and its use as a bioactive substance**

(71) Applicant: BOEHRINGER MANNHEIM GMBH, 68298 Mannheim (DE)
(72) Inventor: Anke, Heidrun, Dr., D-67663 Kaiserslautern (DE); Stadler, Marc, Dr., D-67705 Stelzenberg (DE); Sterner, Olov, Dr., S-21242 Malmö (SE); Hansske, Fritz, Dr., D-69493 Hirschberg (DE)

(57) **Abstract**

This invention relates to a new biological active compound and its derivatives shown as (I). 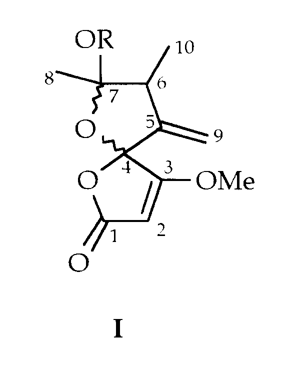 whereas R is a) a hydrogen (Ia) or b) R represents a branched or unbranched Alkylgroup (Ib). More particularly, this invention relates to papyracillic acid (Ia), derivatives (Ib) and salts thereof especially their pharmaceutical acceptable salts, to process of preparation thereof and to bioactive, preferred pharmaceutical, compositions comprising the same.

## Description

This invention relates to a new biological active compound and its derivatives shown as (I). 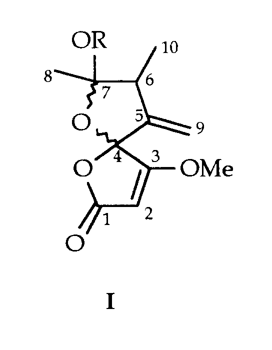 whereas R is a) a hydrogen (Ia) or b) R represents a branched or unbranched C₁-C₄-Alkylgroup (Ib). For R=H, the compound called papyracillic acid.

More particularly, this invention relates to papyracillic acid (Ia), derivatives (Ib) and salts thereof especially their pharmaceutical acceptable salts, to process of preparation thereof and to bioactive, preferred pharmaceutical, compositions comprising the same. Further derivatives of papyracillic acid are obtained by reaction of (Ia) with alkylation agents.

Prodrugs of papyracillic acid and its derivatives are included in this invention. For isolation and purification, salts which are not pharmaceutical safe can be used as well.

The compounds (I) and their derivatives can be solvated, especially hydrated. Hydration may happen during preparation or storage.

Compounds I and their derivatives show different asymmetric centres. The invention includes racemates and optically active forms of papyracillic acid. In addition it was found that papyracillic acid shows antibiotic activity.

The invention includes fermentation fluids, extracts, and concentrated solutions which contain papyracillic acid or its derivatives.

Papyracillic acid can be produced by culturing a papyracillic acid producing strain, e.g. Ascomycete *Lachnum papyraceum* (Karst.) Karst. of the genus Ascomycetes in a nutrient medium containing CaBr_{2.}

A related compound is Penicillic acid which is a classical mycotoxin produced by various fungi including the genera *Penicillium* and *Aspergillus*. Together with patulin, isopatulin and ascladiol it constitutes a class of chemically relatively simple 5-membered cyclic lactones, which due to their toxicity and carcinogenicity are considered to be a potential health hazard to animals and man. [R. J. Cole, R. H. Cox, Handbook of Toxic Fungal Metabolites, p. 510-526, Acadamic Press, New York, 1981]

The micro-organism which can be used for the production of papyracillic acid is a papyracillic acid producing strain belonging to the genus Ascomycetes. Morphological, biological, physiological and cultural characteristics of Ascomycetes *Lachnum papyraceum* can be found in [Dennis, R.W.G.. A revision of British Hyaloscyphaceae with notes on related European species. Mycol. Papers 32 Kew 1949; Karsten, P.A.; Mycologia Femica. Parsprima. Discomycetes Bildrag till Kännedom of Finnlands Natural Folk, Helsingtors, pp. 1-263, 1871]. Synonyms used for *Lachnum papyraceum* are: *Lachnum* (syn. Dasyscyphella, Dasyscyphus, Dasyscypha) *papyraceum* (syn. papyraceus) (Karst.) P. Karst. [Ainsworth, G.C., Sparrow, F.K. & Sussman, A.S. (eds.) The fungi. An advanced treatise Vol IV A: A taxonomic review with keys: Ascomycetes and Fungi Imperfecti. Academic Press New York/London (1976), p. 297].

Ascomycetes *Lachnum papyraceum*, strain 48 ∼ 88, was collected in 1988 in Hinterstein, Germany. A voucher specimen, which showed the characteristics of the genus and species according to Dennis and Karsten, and strain A 48 ∼ 88 (obtained from the ascospores) are deposited in the herbarium and the culture collection of the Lehrbereich Biotechnology, University of Kaiserslautern. It is deposited as well with DSM, Deutsche Sammlung von Mikroorganismen und Zellkulturen GmbH, Mascheroder Weg 1b, D-38124 Braunschweig, Germany, under accession number DSM 10201 (the deposition date is August 3, 1995).

It is to be understood that the production of papyracillic acid is not limited to the use of the particular organism described herein, which is given for the illustrative purpose only. This invention also includes the use of any mutants which are capable of producing papyracillic acid including natural mutants as well as artificial mutants which can be produced from the described organism by conventional means such as irradiation of X-ray, ultra-violet radiation, treatment with N-methyl-N'-nitro-N-nitrosoguanidine, 2-aminopurine, and the like.

For growth of Ascomycetes *Lachnum papyraceum* and therefore production of papyracillic acid, all suitable culturing methods can be used resulting in production of sufficient bio mass. In general, seeding of Ascomycetes *Lachnum papyraceum* and fermentation to papyracillic acid is independent of used container, fermentors and starter proceedings.

Fermentation of the fungus was sucessful in MGP medium [Example 1, Lit. Stadler et al. J. Antibiotics 48, 149-154, 1995] with 100 mM calcium bromide, but other culture media like BAF medium [Singer, R. The Agaricales in modem taxonomy. Springer Verlag Berlin/Heidelberg/New York 1976), YMG media (glucose 0,4% or 1% respectively; malt extract 1,0%, yeast extract 0,4%, and malt extract medium (malt extract 0,5 - 5%) were also suitable for the production of papyracillic acid. The production of compounds I was also observed in Potato Dextrose broth, CMG broth, Czapek-Dox broth (with glucose or sucrose respectively) and cornmeal medium (if not specified otherwise, these culture media are described in the ATCC Media Handbook, American Type Culture Collection Rockville Md, USA 1984).

Assays for antibiotic activity of the extracts of fermentations to which 100 mM CaBr₂ was added at the onset of the secondary metabolism indicated that strongly active metabolites are formed during these conditions, and TLC analyses show that a new product that has not been observed during previous fermentations of the fungus is formed in large amounts. The new product (I) was obtained by silica gel chromatography, and spectroscopic characterisation by NMR suggested that it is a mixture of four isomers (approximately 1:1:2:4 in chloroform according to the ¹H NMR spectrum).

Preferred fermentation conditions and media are given in example 1.

### Derivatives of papyracillic acid

Derivatives of papyracillic acid with R=C₁-C₄-Alkyl are included in this invention.

These compounds can be generally obtained by reaction of compound (Ia) with appropriate alkylation agents.

The methylation of papyracillic acid (Ia) with Trimethylsilydiazomethane (TMSCHN₂) in Benzene/Methanol 1:1 yielded an unexpected number of products (cf. Example 4-6).

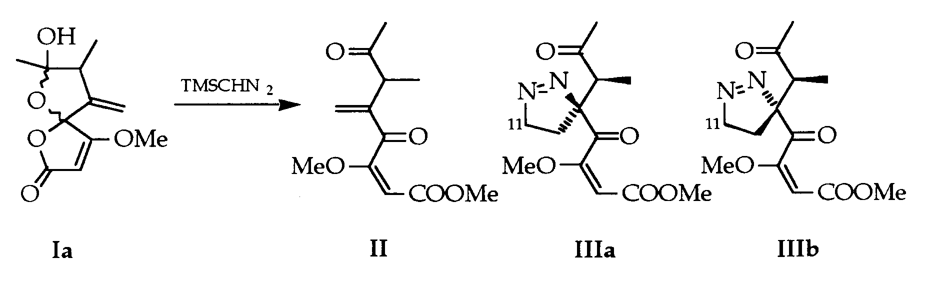

The methyl ester II is formed rapidly, and is the major product as long as reaction time is short (minutes). In addition, the two azo derivatives IIIa) and IIIb) were formed together with the ester II. The formation of similar cyclic azo products (via a 1,3-dipolar cycloaddition of the reagent to the double bond) when α,β-unsaturated carbonyl compounds are treated with diazomethane or TMS-diazomethane has been reported, although these normally are oxidised to pyrazoles or rearranged to pyrazolines.[Aoyama, T.; Iwamoto, Y.; Nishigaki, S.; Shiori, T. Chem. Pharm. Bull. 1989, 37, 253-256].

### Biological activity of papyracillic acid

Papyracillic acid shows interesting pharmaceutical properties in different test systems. For example antibacterial activity (≤ 10 µg/ml) was found against *Bacillus brevis*, *Bacillus substilis, Micrococcus luteus* and *Enterobacter dissolvens*. Antifungal activity is observed with less or about 5 µg/ml against *Nematosopra coryli*. It is less active (10 µg/ml) with *Mucor miehei*, *Penicillium notatum*, *Paecilomyces varioti*. The cytotoxic activity (IC₉₀) is determined to be in the rage of 2-5 µg/ml.

The plate diffusion test was performed as described in "Biology of Antibioties", Springer Verlag, N.Y. 1972. The nutrient broth for bacteria was obtained from DIFCO. The growth medium for fungi and yeast contained 4 g yeastextract, 10 g Maltose, 4 g Glucose and 20 g Agar per 1 l water.

Pharmaceutical compositions of papyracillic acid. In order to produce pharmaceutical agents, the compounds of the general formula I are mixed in a know manner with suitable pharmaceutical carrier substances, aromatics, flavourings and dyes and are formed for example into tablets or coated tablets or they are suspended or dissolved in water or an oil such as e.g. olive oil with addition of appropriate auxiliary substances.

The substance of the general formula I can be administered orally or parenterally in a liquid or solid form. Water is preferably used as the injection medium which contains the stabilizing agents, solubilizers and/or buffers which ar usually used for injection solutions. Such additives are for example tartrate or borate buffers, ethanol, dimethylsulfoxide, complexing agents (such as ethylenediaminetetraacetic acid), high molecular polymers (such as liquid polyethylene oxide) for the regulation of the viscosity or polyethylene oxide) for the regulation of the viscosity or polyethylene derivatives of sorbitol ahydrides.

Solid carrier substances are e.g. starch, lactose, manitol, methylcellulose, talcum, highly dispersed silicic acid, higher molecular fatty acids (such as stearic acid), gelatin, agar-agar, calcium phosphate, magnesium stearate, animal and vegetable fats or solid high molecular polymers (such as polyethylene glycols). Suitable formulations for the oral application can if desired contain flavourings and sweeteners.

The administered dose depends on the age, the health and the weight of the recipient, the extent of the disease, the type of treatments which are possibly being carried out concurrently, the frequency of the treatment and the type of the desired effect. The daily dose of the active compound is usually 0.1 to 50 mg/kg body weight. Normally 0.5 to 40 and preferably 1.0 to 20 mg/kg/day in one or several applications per day are effective in order to obtain the desired results.

### Structure of papyracillic acid and its derivatives

The structure determination of papyracillic acid (Ia) and its derivatives is based on 2D NMR experiments, and pertinent HMBC correlations for compounds Ia, II and IIIa) are presented below. No molecular ion could, as expected, be observed in the EI or CI mass spectra of the two azo derivatives IIIa and IIIb, however, by decreasing the temperature of the ion source from 250 °C to 110 °C the ion M+NH₄⁺ (*m/z* 300) was approximately as abundant as the M-N₂+NH₄⁺ (*m/z* 272) in the CI (NH₃) mass spectra of both compounds. In addition, the ions for M₂+NH₄⁺ (*m/z* 582), M₂-N₂+NH₄⁺ (*m/z* 554) and M₂-N₄+NH₄⁺ (*m/z* 526) became stronger (3-5 % of the base peak). The relative stereochemistry of IIIa and IIIb was suggested by the NOESY correlations observed and the chemical shifts of the C-6 methyl groups in the ¹H NMR spectrum. 10-H₃ correlate strongly to one of protons of C-9 and one of C-11 in both compounds, while 8-H₃ do not, suggesting that the C-6 methyl group is positioned above the five-membered ring in the most stable conformation of the two compounds. This is further supported by the weaker NOESY correlation observed between the C-3 methoxy protons and 6-H. The chemical shift for 10-H₃ is shifted upfield with 0.4 ppm in compound IIIa compared to compound IIIb, and this could be explained by the stronger anisotropic effect of the azo function on 10-H₃ of compound IIIa. 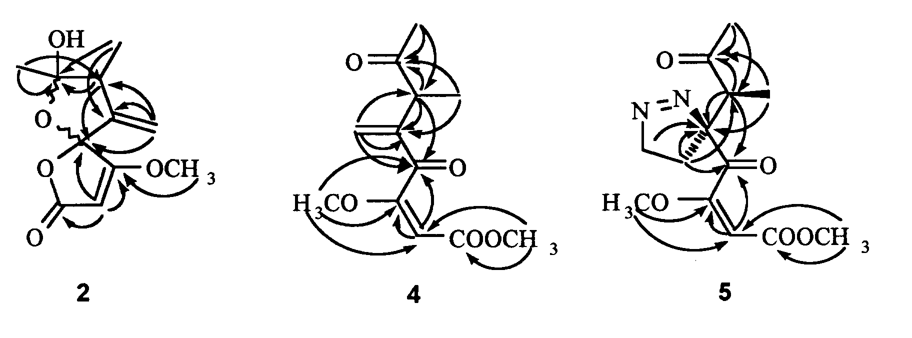 Pertinent ¹H-¹³C long-range correlations observed for the papyracillic acid (**Ia**) and its derivatives **II** and **IIIa**.

The following examples are given for the purpose of illustrating this invention.

### Example I

Strain A 48 ∼ 88 of *Lachnum papyraceum* was maintained and cultivated on MGP medium (maltose 2%, glucose 1%, soypeptone 0.1%, yeast extract 0.1%, KH₂PO₄ 0.1%, MgSO₄ 0.005%, CaCl₂ xH₂O 10mM, FeCl₃ 6µM, ZnSO₄ 7H₂O 6µM) and in the presence of 50-500 mM CaBr₂. Fermentations were carried out in a 20-liter fermentor (Braun Biostat U) at 24°C with an aeration rate of 3.2 liters/minute and agitation (140 rpm). Oxygen saturation of the culture broth was measured using a Braun Oxygen electrode. Aliquots of the culture fluid (100ml) were extracted twice with ethyl acetate. The combined extracts were dried with Na₂SO₄. An extract of *Lachnum papyraceum* was dissolved in methanol (50 ml), and subjected to flash chromatography on a silica gel column eluted with ethyl acetate/heptane 1:1. The fractions were analysed by TLC on silica gel plates in toluene:acetone 7:3, the Rf value of papyracillic acid in this system is 0.60 and upon spraying the plate with anisyl aldehyde/sulfuric acid it gives a deep-green coloured spot. The fractions containing papyracillic acid were purified once more in the same system (silica gel column eluted with ethyl acetate: heptane 1:1) whereafter pure papyracillic acid was obtained from recrystallisation in methanol:water 1:5. The NMR spectra were recorded with a Bruker ARX500 spectrometer, the UV spectra with a Perkin Elmer λ16, the IR spectra with a Bruker IFS48, and the mass spectra with a Jeol SX102 spectrometer.

### Example 2

Papyracillic acid (Ia) was obtained as white crystals, m.p. 97-99°C. [α]_{D} 0° (c 1.0 in methanol). UV (methanol) λₘₐₓ (ε): 226 nm (6,200). IR (KBr): 3450, 2920, 1770, 1640, 1360, 1210, 940 and 860 cm⁻¹. ¹H NMR (500 MHz in CDCl₃), δ, mult. *J* (Hz): 5.23-5.05, 2-H and 9-H₂; 3.89, 3.85, 3.84 and 3.83, 4s, 3-OCH₃; 2.89, dm, J₆₋₁₀=7.2; 2.84, 2.75 and 2.66, ddq, J₆₋₉ₐ=3, J_{6-9b}=3, J₆₋₁₀=7, 6-H; 1.57, 1.54, 1.38 and 1.36, 4s, 8-H₃; 1.14, 1.13, 1.13 and 1.10, 4d, J₆₋₁₀=7, 10-H₃. ¹³C NMR (125 MHz in CDCl₃), δ: 178.2, 177.9, 176.7 and 176.2 C-3; 170.4, 170.3, 170.2 C-1; 149.4, 148.3, 148.2 and 147.8 C-5; 111.4, 111.3, 111.2 and 110.9 C-9; 109.5, 109.2, 107.3 and 107.3 C-7; 107.3, 107.3, 107.1 and 106.2 C-4; 91.1, 90.2, 88.8, 88.4 C-2; 60.1, 60.0, 60.0, 59.8 OCH₃; 47.9, 47.4, 47.0 and 45.1 C-6; 25.1, 24.4, 22.6 and 22.4 C-8; 15.2, 12.7, 10.9 and 10.4 C-10. MS (EI, 70 eV), *m/z*: 209.0791 (M⁺ - OH, 100 %, C₁₁H₁₃O₄ requires 209.0814), 184 (12 %), 166 (56 %), 139 (29 %), 123 (13 %), 69 (18 %), 43 (24 %).

### Example 3

The reaction of Ia yielding Acetals is performed in a usual maner by katalyzing with acid. The methylated compound Ib (R=CH₃) was obtained by stirring at room temperature leaving Papyracillic acid (Ia) in Methanol with traces of trifluoracetic acid present

A mixture of isomers was obtained, from which compound Ib could be isolated as the major isomer. White crystals, m.p. 116-118°C. [α]_{D} -42° (c 1.3 in chloroform). UV (methanol) λₘₐₓ (ε): 224 nm (9,800). IR (KBr): 2940, 1770, 1640, 1460, 1360, 1210, 1075, 950 and 870 cm⁻¹. ¹H NMR (500 MHz in CDCl₃), δ, mult. *J* (Hz): 5.12, d, J₆₋₉ₐ=3, 9-HA; 5.10, d, J_{6-9b} =3, 9-Hb; 5.01, s, 2-H; 3.81, s, 3-OCH₃; 3.23, s, 7-OCH₃, ddq, J₆₋₉ₐ =3, J_{6-9b} =3, J₆₋₁₀=6.8, 6-H; 1.43, s, 8-H₃, 1.08, d, J₆₋₁₀=6.8, 10-H₃. ¹³C NMR (125 MHz in CDCl₃), δ: 178.0 C-3; 170.1 C-1; 148.5 C-5; 109.6 C-9; 109.1 C-7; 106.8 C-4; 88.1 C-2; 59.6 3-OCH₃; 49.2 7-OCH₃; 48.5 C-6; 18.7 C-8; 10.3 C-10. MS (EI, 70 eV), m/z: 209.0788 (M+ - OCH₃, 54 %, C₁₁H₁₃O₄ requires 209.0814), 166 (100 %), 151 (29 %), 123 (31 %), 69 (39 %), 43 (43 %).

### Example 4

Methyl papyracillate (II) was obtained as a colourless oil. [α]_{D} +25 ° (c 1.0 in chloroform). UV (methanol) λₘₐₓ (ε): 224 nm (11,700). IR (KBr): 2950, 1715, 1685, 1620, 1370, 1195, 1145 and 1020 cm⁻¹. ¹H NMR (500 MHz in CDCl₃), δ, mult. *J* (Hz): 6.00 and 5.95, 2s, 9-H₂; 5.23, s, 2-H; 3.82, q, J₆₋₁₀=7.2, 6-H; 3.70, s, 3-OCH₃; 3.56, s, 1-OCH₃; 2.15, s, 8-H₃; 1.19, d, J₆₋₁₀=7.2, 10-H₃. ¹³C NMR (125 MHz in CDCl₃), δ: 207.8 C-7; 191.0 C-4; 167.2 C-3; 166.2 C-1; 146.1 C-5; 129.2 C-9; 93.1 C-2; 56.8 3-OCH₃; 51.1 1-OCH₃; 45.4 C-6; 28.4 C-8; 15.1 C-10. MS (EI, 70 eV), *m/z*: 240.0976 (M⁺, 20 %, C₁₅H₂₂O₂ requires 240.0998), 208 (42 %), 198 (99 %), 166 (60 %), 139 (100 %), 123 (29 %), 69 (42 %), 43 (73 %).

### Example 5

Compound IIIa was obtained as white crystals, m.p. 97-99°C, in 10 % yield after methylation of papyracillic acid (Ia) with TMS-diazomethane in methanol: benzene 1:1 at room temperature for 5 h. [α]_{D} +43 ° (c 0.6 in chloroform). UV (methanol) λₘₐₓ (ε): 222 nm (7,800). IR (KBr): 2920, 1710, 1700, 1620, 1445, 1360, 1200, 1140, 1070 and 815 cm⁻¹. ¹H NMR (500 MHz in CDCl₃), δ, mult. *J* (Hz): 5.14, s, 2-H; 4.66, ddd, J₉ₐ₋₁₁=6.4, J_{9b-11a}=10.2, J_{11a-11b}=18.2, 11-Ha; 4.56, ddd, J_{9a-11b}=9.9, J_{9b-11b}=5.7, J_{11a-11b}=18.2, 11-Hb, 4.01, q, J₆₋₁₀=7.0, 6-H; 3.80, s, 3-OCH₃; 3.57, s, 1-OCH₃; 2.23, s, 8-H₃; 2.12, ddd, J_{9a-9b}=13.9, J₉ₐ₋₁₁ₐ=6.4, J_{9a-11b}=9.9, 9-Ha; 2.04, ddd, J_{9a-9b}=13.9, J_{9b-11a}=10.1, J_{9b-11b}=5.7; 0.79, d, J₆₋₁₀=7.0, 10-H₃. ¹³C NMR (125 Mhz in CDCL₃), δ: 208.5 C-7; 197.7 C-4; 167.5 C-3; 166.9 C-1; 105.2, C-5; 93.0 C-2; 79.1 C-11; 57.4 3-OCH₃; 51.6 1-OCH₃; 47.3 C-6; 31.2 C-8; 20.3 C-9; 11.2 C-10. MS (EL 70 eV), m/z: 254.1137 (M⁺-N₂, 5 %, C₁₃H₁₈O₅ requires 254.1154), 222 (8 %), 212 (32 %), 211 (19 %), 179 (17 %), 153 (100 %), 137 (18 %), 111 (21 %), 69 (26 %), 43 (50 %). MS (CI, NH₃, ion source temperature 250°C ), m/z: 272 (M-N₂+NH₄⁺, 100 %), 255 (M-N₂+H⁺, 25%), 237 (33 %). MS (CI, NH₃, ion source temperature 110°C), m/z: 300 (M+NH₄⁺, 100 %), 283 (M+H⁺, 3 %), 272 (M-N₂+NH₄⁺, 82 %), 255 (M-N₂+H⁺, 13 %), 237 (32 %).

Compound 6 was obtained as a colourless oil in 10% yield after methylation of papyracillic acid (2a) (vide supra).

### Example 6

Compound IIIb was obtained as a colourless oil in 10 % yield after methylation of papyracillic acid (Ia) (*vide supra*). [α]_{D} +10 ° (c 0.5 in chloroform). UV (methanol) λₘₐₓ (ε): 222 nm (8,400). IR (KBr): 2920, 1700, 1615, 1435, 1360, 1190 and 1140 cm⁻¹. ¹H NMR (500 MHz in CDCl₃), δ, mult. *J*(Hz): 5.15, s, 2-H; 4.66, ddd, J₉ₐ₋₁₁ₐ=6.3, J_{9b-11a}=8.5, J_{11a-11b}=18.1, 11-Ha; 4.63, ddd, J_{9a-11b}=7.9, J_{9b-11b}=7.0, J_{11a-11b}=18.1, 11-Hb, 4.03,q, J₆₋₁₀=7.2, 6-H; 3.84, s, 3-OCH₃; 3.55, s, 1-OCH₃; 2.13, s, 8-H3; 2.07, ddd, J_{9a-9b}=12.7, J₉ₐ₋₁₁ₐ=6.3, J_{9a-11b}=7.9, 9-Ha; 2.03, ddd, J_{9a-9b}=12.7, J_{9b-11a}=8.5, J_{9b-11b}=7.0; 1.17, d, J₆₋₁₀=7.2, 10-H₃ ¹³C NMR data were not recorded. MS (EI, 70 eV), m/z: 254.1159 (M⁺-N₂, 7%, C₁₃H₁₈O₅ requires 254.1154), 223 (12 %), 212 (38 %), 211 (59 %), 179 (30 %), 153 (100 %), 137 (28 %), 111 (37 %), 69 (38 %), 43 (72 %). MS (CI, NH₃, ion source temperature 250°C), m/z: 272 (M-N₂+NH₄⁺, 100 %), 255 (M-N₂+H⁺, 27 %), 237 (34 %). MS (CI, NH₃, ion source temperature 110°C), m/z: 300 (M+NH₄⁺, 100 %), 272 (M-N2+NH4+, 85 %), 255 (M-N₂+H⁺, 8 %), 237 (23 %).

## Claims

1. Compounds having the formula (I) 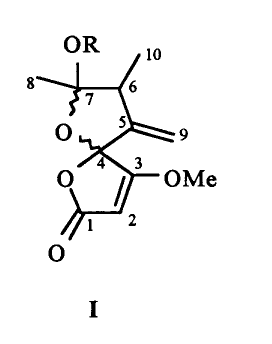 whereas R represents a hydrogen or a branched or unbranched C₁-C₄-Alkylgroup their optically active forms, and their pharmacologically safe salts.

2. Process for preparation of compounds I of claim 1 which comprises culturing of a strain belonging to genus Ascomyces which produces substances according to claim 1 in a nutrient medium containing in addition CaBr₂.

3. A process as claimed in claim 2, wherein the substance producing strain is Ascomecetes *Lachnum papyraceum* (Karst.) Karst. strain 44 ∼ 88.

4. Compounds obtainable by a process according to one of the claims 3 or 4.

5. A pharmaceutical composition comprising active ingredients according to claim 1, their optically active isomers, their pharmaceutically acceptable salts, or a prodrug of said active ingredients.

6. Use of compounds according to are of the claims 1 or 4 for production of drugs, with antibacterial, antifungiacal or cytocoxic activity.
